# EUROPEAN PATENT APPLICATION

(11) **EP 2 499 974 A1**
(43) Date of publication of application: **19.09.2012**
(21) Application number: 10829945.4
(22) Date of filing: 10.11.2010
(51) Int. Cl.: A61B 8/00

(54) **ULTRASONIC DIAGNOSTIC SYSTEM**

(30) Priority: 10.11.2009 JP 2009256886; 10.11.2009 JP 2009256887
(71) Applicant: The University of Tokyo, Bunkyo-Ku, Tokyo 113-8654 (JP); Hitachi Aloka Medical, Ltd., Mitaka-shi Tokyo 181-8622 (JP)
(72) Inventor: KUBOTA, Naoto, Tokyo 113-8654 (JP); KOIZUMI, Norihiro, Tokyo 113-8654 (JP); LIAO, Hongen, Tokyo 113-8654 (JP); ASANO, Takaharu, Tokyo 113-8654 (JP); YUHASHI, Kazuhito, Tokyo 113-8654 (JP); MITSUISHI, Mamoru, Tokyo 113-8654 (JP); OHNISHI, Shin, Tokyo 113-8654 (JP); MOCHIZUKI, Takashi, Mitaka-shi Tokyo 181-8622 (JP); SAKUMA, Ichiro, Tokyo 113-8654 (JP); KADOWAKI, Takashi, Tokyo 113-8654 (JP)
(74) Representative: Heim, Florian Andreas
(86) International application number: PCT/JP2010/069976
(87) International publication number: WO 2011/058986

(57) **Abstract**

When an amount of visceral fat is measured using ultrasonic waves during medical examination of metabolic syndrome, three contact positions are defined on the surface of the abdominal region, and three distances inside the living body are measured by bringing a probe into contact with the surface of the abdominal region at the contact positions. Specifically, three measurement paths having a starting point at the center of the descending aorta are set, and the distances from the center to a surface of the fat layer adj acent to the surface of the body (inner surface of the subcutaneous layer) in the measurement paths are observed. An approximate area of a visceral fat region (20) can be determined from the three distances and two angles defined by the three measurement paths, and an index value is calculated on the basis of the area. A probe holder is desirably used during the above-described measurement. The probe holder includes three holding portions that selectively hold the probe.

## Description

### TECHNICAL FIELD

The present invention relates to an ultrasonic diagnostic system, and in particular to an ultrasonic diagnostic system which measures visceral fat.

### BACKGROUND ART

In the medical field, ultrasonic diagnostic systems are being utilized. An ultrasonic diagnostic system is generally formed from an ultrasonic diagnostic device or from a combination of an ultrasonic diagnostic device and a computer. The ultrasonic diagnostic device comprises an ultrasonic probe which transmits ultrasound to a living body and receives a reflected wave from the living body, and a device body which executes image formation and various measurements based on the received signal from the ultrasonic probe. With the ultrasonic diagnosis, it is possible to avoid a problem occurring in X-ray diagnosis such as radiation exposure, and the ultrasonic diagnosis does not require a large-scale device such as that required for X-ray diagnosis. Because of such convenience, it is desired to apply the ultrasonic diagnostic technique to medical examination of metabolic syndrome (that is, obesity due to visceral fat).

Currently, in medical examination of metabolic syndrome, in general, abdominal circumference is measured, because there is a certain degree of correlation between the abdominal circumference and the amount of visceral fat. However, abdominal circumference is merely length information including the subcutaneous fat (including muscle), and does not directly represent the amount of visceral fat in the abdominal cavity or the size of the range where the visceral fat exists. A method is proposed in which a weak current is applied to the abdominal region and the amount of visceral fat is estimated based on the electrical resistance thereof. However, for realization of such a method, a large-scale device would be required, and a result which sufficiently reflects the structure in the abdominal region cannot be obtained, and, thus, the reliability of measurement cannot be improved. In a method of measuring the amount of visceral fat using an X-ray CT device, measurement with high precision can be realized, but a very large-scale system must be constructed for this method, resulting in problems in scale and cost. Inaddition, a problem arises in relation to radiation exposure. In consideration of this, research has been carried out on application of ultrasonic diagnosis, which can non-invasively observe the in-body structure, to medical examination of metabolic syndrome; that is, visceral fat measurement.

Non-Patent Literature 1 is a paper describing a relationship between visceral fat and cardiovascular disease risk factors. Although the details are not clear, it can be deduced that the amount of visceral fat is measured using an ultrasonic image. More specifically, on a lateral cross section of the abdominal region (cross section vertically crossing the lumber vertebra) shown in FIG. 1 of Non-Patent Literature 1, three paths radially spreading from the lumber vertebra to the front surface side of the abdominal region are set, distances a, b, and c from the lumber vertebra to the subcutaneous fat are determined on these paths, and an average value ((a + b + c)/3) is calculated as information VFD (visceral fat distance) corresponding to the amount of visceral fat. In this calculation, the angle between adjacent paths is not taken into consideration. In other words, in this method, only distance information of one dimension is used, and two-dimensional information or structural information is not used. This paper also fails to disclose a device for setting the three paths with superior reproducibility.

Patent Literature 1 discloses a visceral fat obesity examination device which calculates a ratio of a cross-sectional area of the subcutaneous fat and a cross-sectional area of preperitoneal fat by an image process on an ultrasonic image. However, this device is not targeted to measure a wide range within the abdominal region, and does not have a measurement condition and a measurement support device for realizing superior reproducibility.

Patent Literature 2 discloses a visceral fat measurement device which identifies a preperitoneal fat thickness near the liver and a preperitoneal fat thickness near the navel, and determines a visceral fact coefficient which depends on the amount of visceral fat based on these pieces of information. This device observes the visceral fat at two points distanced in the direction of extension of the spine, and does not take into consideration a shape and a structure in a cross section perpendicular to the spine.

Patent Literature 3 discloses an attachment for ultrasonic probe, whichprevents a change of the fat thickness when the ultrasonic probe comes into contact with the patient. However, this attachment only has one probe-holding portion. Patent Literature 4 discloses a near-infrared light type body fat measurement device having a band-shapedstring. On the string, a navel position matching portion is provided.

In the examination of metabolic syndrome; in particular, in a group medical examination of metabolic syndrome, easy, quick, and reliable measurement of the information corresponding to the amount of visceral fat is desired. However, with the techniques of the related art, such desire cannot necessarily be sufficiently satisfied.

In the case of examination by contacting the probe in a plurality of positions on a body surface in sequence, improvement of the positioning precision of the probe and realization of superior operability at the positioning of the probe are desired, but with the technique of related art, such desires cannot necessarily be sufficiently satisfied.

### [Related Art References]

### [Patent Literature]

[Patent Literature 1] JP 2007-135980 A
[Patent Literature 2] JP 2008-194240 A
[Patent Literature 3] JP 2008-284136 A
[Patent Literature 4] JP 2006-296770 A

### [Non-Patent Literature]

[Non-Patent Literature 1] Yu CHIBA et al., "Relationship between Visceral Fat and Cardiovascular Disease Risk Factors: The Tanno and Sobetsu Study", Hypertens Res, Vol. 30, No. 3, 2007, pp. 229-236.

### DISCLOSURE OF INVENTION

### [Technical Problem]

An advantage of the present invention lies in provision of an ultrasonic diagnostic system which can measure information corresponding to the amount of visceral fat with high precision using ultrasound.

Another advantage of the present invention lies in provision of an ultrasonic diagnostic system which can set a plurality of visceral fat measurement paths with superior reproducibility on a cross section of a living body.

Another advantage of the present invention lies in provision of an ultrasonic diagnostic system which can obtain a reliable measurement result with a simple structure and suited for group medical examination of metabolic syndrome.

Another advantage of the present invention lies in provision of an ultrasonic diagnostic system which can improve positioning precision and a positioning reproducibility of the probe on a surface of the living body.

Another advantage of the present invention lies provision of an ultrasonic diagnostic system which can improve operability during positioning of the probe on a surface of the living body.

Another advantage of the present inventions lies provision of an ultrasonic diagnostic system which can easily search a reference tissue which extends in a direction of a body axis and which can construct a superior measurement situation.

### [Solution to Problem]

The invention described in each of the claims of the present application is directed to realizing one of the above-described advantages.

According to one aspect of the present invention, there is provided an ultrasonic diagnostic system comprising an ultrasonic probe which is brought into contact with a livingbody, which transmits and receives ultrasound, and which outputs a reception signal; an image formation unit which forms an ultrasonic image based on the reception signal; a distance measurement unit which uses a plurality of ultrasonic images including a plurality of measurement paths which are radially set on a cross section of the living body, to measure a distance between a reference site at a deep position and a predetermined boundary surface at a shallow position on each of the measurement paths; an index value calculation unit which calculates an index value having a correlation to an amount of visceral fat based on a relative positional relationship among the plurality of measurement paths and a plurality of distances measured on the plurality of measurement paths; and an output unit which outputs the index value.

According to the above-described structure, a range where there is a possibility that the visceral fat exists in the living body is identified as a plurality of distances along a plurality of measurement paths. Based on the relative positional relationship among the plurality of measurement paths (preferably, intersection angles) and the plurality of distances, an index value having a correlation to the amount of visceral fat is calculated. In the abdominal circumference measurement method which is the method of related art, the subcutaneous fat and the thickness of the muscle are also included as measurement targets, but with the above-described configuration, the range where the visceral fat may exist can be identified while removing the subcutaneous fat layer or the like, and use the range as a basis for the index value calculation. More specifically, according to the method disclosed in the present application, a plurality of distances are measured along a plurality of measurement paths, and, thus, a two-dimensional spreading or a size of the visceral fat can be considered. Because of this, the reliability of the index value can be improved. When the X-ray CT device is used for measurement of the visceral fat, a problem of radiation exposure occurs and a large-scale mechanism is required. However, with the above-described configuration, the index value can be measured quickly and non-invasively, and a high level of medical usability can be achieved. The predetermined boundary surface is a boundary surface surrounding an area where the visceral fat exists. Preferably, the predetermined boundary surface is an inner surface of a subcutaneous layer in which the visceral fat does not exist; more specifically, is an inner surface of a muscle layer or an inner surface of a subcutaneous fat layer.

The number of measurement paths is greater than or equal to two, and is preferably three. By setting three measurement paths, in addition to the spreading of the range where the visceral fat may exist, the approximate shape (or a difference between a form on a right side and a form on a left side) of the range canbe considered. Alternatively, four or more measurement paths may be set. The distance measurement is executed automatically, manually, or semi-automatically. In the case of manual execution, in consideration of the user's burden, it is preferable to set three measurement paths. The plurality of measurement paths are preferably set such that the plurality of measurement paths intersect each other at a deep portion in the body. The shape of a range or a body cavity in which the visceral fat may exist is approximately elliptical, and, therefore, it is particularly preferable to set the reference site near the center of the ellipse and set a plurality of measurement paths which radially spread from the reference site. In order to realize the distance measurement on the plurality of measurement paths, the probe is stepwise or simultaneously brought into contact with a plurality of contact positions on the surface of the living body. Preferably, each of the plurality of ultrasonic images which are displayed includes a line representing the measurement path, and there is provided an input unit with which a user designates the reference site and the predetermined boundary surface on each line.

Preferably, the reference site is a blood vessel, each ultrasonic image corresponding to each of the measurement paths is displayed as a tomographic image, and a distance between the blood vessel and the predetermined boundary surface is measured on each of the tomographic images. Displaying the tomographic image facilitates visual identification of the predetermined boundary surface. In addition, the identification of the blood vessel is also facilitated. Alternatively, the identification of the blood vessel may be automatically executed using an ultrasonic Doppler method. Preferably, the blood vessel is a descending aorta which beats. Such a beating blood vessel can be very easily recognized on the tomographic image, and, by setting the measurement paths with reference to such a blood vessel, the reliability of measurement can be improved even for a manual measurement.

Preferably, the plurality of tomographic images correspond to a plurality of scanning planes which are perpendicular to the cross section and which cross each other on the descending aorta. Preferably, the cross section of the living body is a lateral cross section on an abdominal region of the living body, and a central scanning plane among the plurality of scanning planes is formed at a position avoiding the navel existing in the abdominal region.

Preferably, the plurality of scanning planes include a central scanningplane, aright-side scanning plane, and a left-side scanning plane, and the right-side scanning plane and the left-side scanning plane are set on a right side and a left side of the central scanning plane with substantially the same inclination angle with respect to the central scanning plane. Preferably, the plurality of measurement paths include a central path, a right-side path, and a left-side path, and the index value calculation unit comprises a unit which calculates a right-side portion area of a right-side portion between the central path and the right-side path based on a distance along the central path, a distance along the right-side path, and a right-side angle between the central path and the right-side path; a unit which calculates a left-side portion area of a left-side portion between the central path and the left-side path based on the distance along the central path, a distance along the left-side path, and a left-side angle between the central path and the left-side path; and a unit which calculates the index value using at least the right-side portion area and the left-side portion area.

The calculated area value may be output as the index value without further processing, or a volume value may be determined by calculating area values at various positions of the living body and output as the index value. As the method of area calculation and volume calculation, various methods may be considered. In any case, it is preferable to calculate the plurality of distances along the plurality of radial measurement paths and to calculate the index value on the basis of the two-dimensional shape information in the living body.

Preferably, the ultrasonic diagnostic system further comprises probe-holding equipment. Preferably, the probe-holding equipment comprises a plurality of holding portions which store a probe to be brought into contact with the abdominal region, and a fixing unit which fixes the plurality of holding portions on the abdominal region, and the plurality of holding portions are provided aligned in a left-and-right direction of the abdominal region and with an angle to direct a transmission and reception surface of the probe toward the reference site during use.

With the above-described configuration, the plurality of holding portions are provided with a predetermined positional relationship, and a user can set the probe in the holding portions in order and execute the ultrasonic diagnosis at each position. With such a configuration, because the probe can be quickly and accurately positioned, the burden of the user can be reduced, and superior reproducibility of the measurement can be achieved. The reference site is, for example, a blood vessel positioned in a deep portion in the body, and an orientation of the probe is adjusted such that the scanning plane passes through the reference site. Inparticular, the inclination angle is adjusted. In the state where the probe is inserted in each of the plurality of holding portions, in general, the electronic scanning directions become parallel to each other, and only a rake angle of the scanning plane is adjusted. When the electronic scanning direction and the running direction of the blood vessel serving as the reference site are in a parallel relationship, the plurality of scanning planes may intersect on the central axis of the blood vessel. The number of the holding portions is determined according to the measurement objective, and, for the measurement of the visceral fat, for example, three holding portions are provided. Alternatively, two, or four or more holding portions may be provided. The fixing unit preferably surrounds the entirety of the abdominal region, but, alternatively, other structures may be used.

Preferably, each holding portion has a deformability to permit a rake movement of the probe stored therein. Each holding portion is preferably formed with an elastic, deformable material. A gap may be provided in the holding portion to permit the movement of the probe. The holding portion is preferably formed such that the probe is not dropped even when the user is not holding the probe with a hand. Preferably, the positioning of the scanning plane is executed by the user while viewing the ultrasonic image.

Preferably, the probe includes a one-dimensional array transducer, and each holding portion holds the probe such that an element arrangement direction of the one-dimensional array transducer is parallel to a body axis direction of the living body. Preferably, in each holding portion, there are formed an opening for exposing the transmission and reception surface of the probe to the living body side and a hollow structure which surrounds and holds the probe.

Preferably, the plurality of holdingportions include a central holding portion, a right-side holding portion, and a left-side holding portion, the right-side holding portion and the left-side holding portion are inclined with respect to the central holding portion and the overall holding portions spread in a fan shape, and inclination angles of the right-side holding portion and the left-side holding portion with respect to the central holding portion during use are set between 30 degrees and 50 degrees. Preferably, the fixing unit is a belt-shapedmember wound around the body section. Preferably, a marker which is used for position matching with respect to the navel is provided.

### BRIEF DESCRIPTION OF DRAWINGS

FIG. 1 is a diagram for explaining a measurement method of the present invention, and particularly showing three measurement paths for calculating an index value.
FIG. 2 is a diagram for explaining distance measurement on three tomographic images corresponding to three measurement paths.
FIG. 3 is a diagram for explaining an area calculation based on three distances.
FIG. 4 is a conceptual diagram showing a specific example calculation in an area calculation.
FIG. 5 is a diagram for explaining an example calculation of a right-side portion area and a left-side portion area.
FIG. 6 is a diagram for explaining an area calculation method using a table.
FIG. 7 is a diagram for explaining automatic distance calculation along a measurement path.
FIG. 8 is a diagram showing an ultrasonic diagnostic system having a function to calculate an index value having a correlation to an amount of visceral fat.
FIG. 9 is a flowchart of an example operation of the device shown in FIG. 8.
FIG. 10 is a perspec t ive view for explaining a piece of equipment used for fixing the probe on a plurality of positions on a body surface.
FIG. 11 is a cross sectional diagram of the equipment shown in FIG. 10.
FIG. 12 is a diagram for explaining an overall structure of the equipment shown in FIG. 10

### BEST MODE FOR CARRYING OUT THE INVENTION

A preferred embodiment of the present invention will now be described with reference to the drawings.

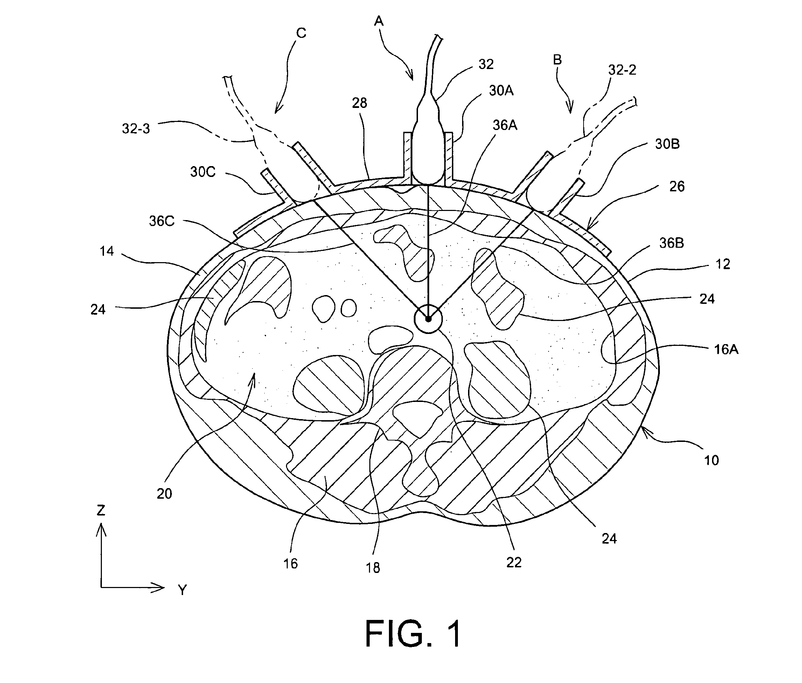

FIG. 1 schematically shows a lateral cross section of an abdominal region of a living body. FIG. 1 particularly shows a situation where an index value having a correlation to the amount of visceral fat is measured and calculated. An X direction is a direction of the spine, a Z direction is a direction of thickness of the living body, and a Y direction is a left-and-right direction. The lateral cross section of FIG. 1 is a cross section which is observed by setting a viewing line from a leg side toward a head side.

In FIG. 1, reference numeral 10 represents an abdominal region of the living body, with a lower side of the abdominal region 10 representing the back and an upper side of the abdominal region 10 representing a surface 12 of the abdominal region 10. For example, the living body is placed on a bed facing upward. In the inside of the abdominal region 10, a subcutaneous fat layer 14 exists. The subcutaneous fat layer 14 is a layer including the skin and the muscle. A muscle 16 exists also in an inner side of the subcutaneous fat layer 14. A visceral fat area 20 is present in a further inner side of the muscle 16. In FIG. 1, the visceral fat area 20 is a gap region extending in the YZ plane, and exists around the organs. The existence percentage differs depending on the person. With the present method described below, a special index value having a significant correlation to the amount of visceral fat can be calculated. A subcutaneous layer may be defined as a layer including the subcutaneous fat layer 14 and the muscle 16. In FIG. 1, the visceral fat area 20 is surrounded by an inner surface (boundary surface) 16A of the subcutaneous layer.

In FIG. 1, reference numeral 18 represents a lumber vertebra, and reference numeral 24 represents other tissues such as an organ. The tissue which should be observed here is a descending aorta 22. The descending aorta is a wide artery, and a beat of the descending aorta can be easily viewed on the ultrasonic image. The descending aorta 22 is positioned at an approximate center of the abdominal region, and, in the present embodiment, the descending aorta 22 is used as a reference tissue or a reference site.

For the measurement of the index value, in the present embodiment, 3 measurement paths 36A, 36B, and 36C are set. In FIG. 1, these paths are shown as 3 lines, and these lines cross each other at a center of the descending aorta 22. With respect to the measurement path 36A at the center, the other two measurement paths 36B and 36C are inclined by the same angle. The angle is, for example, 40 degrees. The angle value may alternatively be set in a range of 30 degrees to 50 degrees or to another angle. Three contact positions A, B, and C are determined, in order to set the three measurement paths 36A, 36B, and 36C radially.

More specifically, a piece of holding equipment 26 is provided on the abdominal region surface 12, and a probe 32 is sequentially held at the three contact positions A, B, and C by the holding equipment 26. The holding equipment 26 has three holding portions 30A, 30B, and 30C, and the probe 32 may be selectively inserted and held in one of the holding portions 30A, 30B, and 30C. For example, in FIG. 1, the probe 32 is provided in the contact position A; that is, the probe 32 is inserted in the holding portion 30A. A transmission and reception surface of the probe 32 is in close contact with the abdominal region surface 12, and ultrasound is transmitted and received in this state; that is, electronic scanning of the ultrasonic beam is executed. When the measurement at this position is completed, the probe 32 is next moved to the contact position B, and a similar ultrasonic measurement is executed at this position. Then, ultrasonic measurement similar to the above is executed at the contact position C. Reference numeral 28 represents a base portion of the holding equipment 26, and reference numerals 32-2 and 32-3 represent the probe after the probe is re-inserted. The electronic scan direction of the probe 32 is in the X direction. That is, the electronic scan is executed in a direction perpendicular to the lateral cross section shown in FIG. 1, and the scanning plane is formed in this direction. The central contact position A is set at a position slightly avoiding a navel position where an air layer gap tends to be generated. With this configuration, superior acoustic propagation is ensured at all times. The contact position A is positioned immediately above the descending aorta 22.

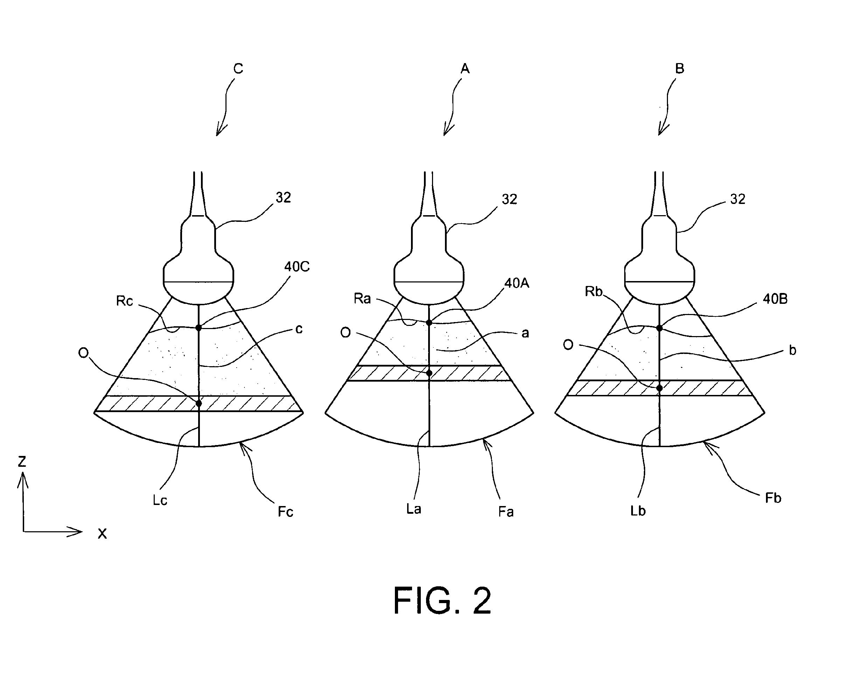

In the present embodiment, in order to measure a value corresponding to the area of the visceral fat area 20 shown in FIG. 1, three measurement paths 36A, 36B, and 36C are set as described above, and, along each of the measurement paths 36A, 36B, and 36C, a distance from the center of the descending aorta to a boundary surface 16A existing on the side of the front surface of the living body is manually or automatically measured. FIG. 2 shows an example configuration where the distance is measured manually. In FIG. 1, the boundary surface 16A is an inner surface of the muscle layer. Alternatively, an inner surface of the subcutaneous fat layer 14 may be used as the reference surface.

In FIG. 2, three tomographic images Fa, Fb, and Fc are shown corresponding to the three contact positions A, B, and C described above. The tomographic images Fa, Fb, and Fc are formed based on echo data on three scanning planes. Here, each scanning plane is formed by electronic scanning of an ultrasonic beam. In FIG. 2, the probe 32 which is placed in each contact position is conceptually shown. In each tomographic image, a line representing the measurement path is displayed.

When, for example, reference is made to the central tomographic image Fa, the measurement path is shown with reference numeral La in this image. When the distance measurement is executed, a center O of the descending aorta is designated by the user, and a point 40A corresponding to a depth position of a boundary surface Ra is designated by the user. These two points O and 40A are designated on the measurement path La corresponding to a central line. Alternatively, there may be employed a configuration in which such a path can be freely inclined or deflected. The boundary surface Ra is in general a surface which can be easily visually identified, and the descending aorta can also be very easily identified on the image. Therefore, the distance can be identified with a high level of precision. Similarly, at the contact position B also, along the measurement path Lb, the center O of the descending aorta and a point 40B on a boundary surface Rb are designated by the user, and a distance b is automatically identified as a result. Similarly, at the contact position C also, along the measurement path Lc, the center point O and a point C on a boundary surface Rc are identified by the user, and a distance c is automatically calculated. With the above-describe process, the three distances a, b, and c are recognized.

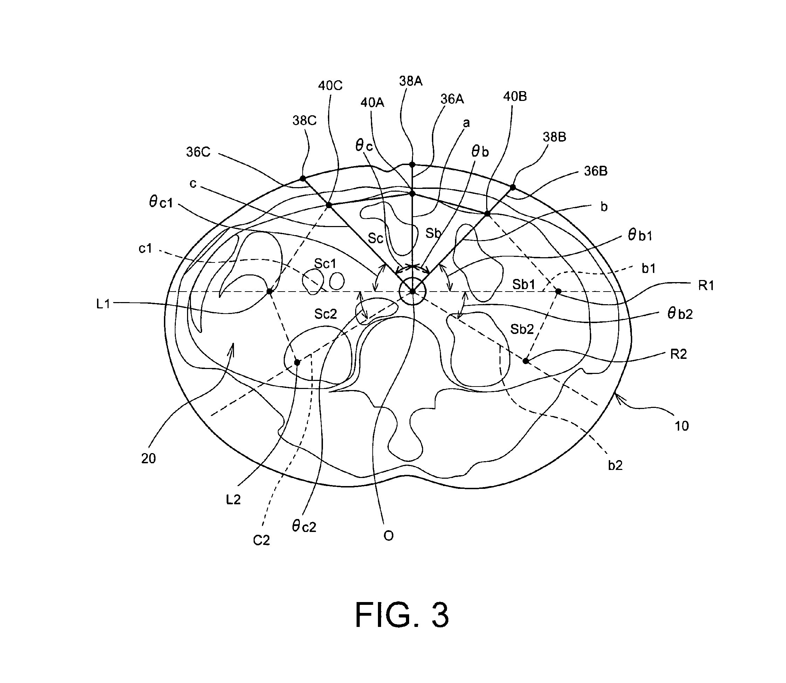

FIG. 3 again shows a lateral cross section of the living body. Points 38A, 38B, and 38C on the measurement paths 36A, 36B, and 36C represent center points on the transmission and reception surface, and O represents the center point of the descending aorta as described above. Reference numerals 40A, 40B, and 40C represent points on the boundary surface designated by the above-described process. In the present embodiment, the central measurement path 36A is set vertical. Inclination angles θb and θc of the other two measurement paths 36B and 36C with respect to the central measurement path are known, and, for example, the inclination angles θb and θc are both 40 degrees. With this process, four points for identifying two triangles are defined. That is, a two-dimensional shape of a quadrangle or two triangles surrounded by four points including the center point O, the boundary point 40B, the boundary point 40A, and the boundary point 40C can be identified. According to the experiments by the present inventors, it has been shown that such a size or area of the two-dimensional shape and the amount of visceral fat in the abdominal cavity are in a strong correlation relationship. Thus, an index value showing the size of the amount of visceral fat can be calculatedusing the size or the area of the two-dimensional shape.

As a method of obtaining such an index value, there exist a function calculation method and a table method. In the following, first, the function calculation method will be described. In this method, the area is calculated from the geometric viewpoint (that is, relative positional relationship among the three measurement paths and three distances). The details will now be described.

Areas Sb and Sc of the two triangles can be easily determined based on the distances a, b, and c which are already calculated, and the 2 angles θb and θc which are known. In the present embodiment, such a method is expanded to further calculate areas of four triangles. That is, partial areas Sb1, Sb2, Sc1, and Sc2 are calculated.

The area Sb1 is an area of a triangle surrounded by the points O, 40B, and R1, and can be calculated from the angle θb1 and lengths b and b1 of two sides. The angle θb1 is a known value, and the length b1 of the side is defined in the present embodiment as the same length as the side b or a length obtained by multiplying the length of side b by a predetermined coefficient. The area Sb2 is an area of a triangle surrounded by 3 points O, R1, and R2, and is calculated based on lengths b1 and b2 of the sides and an angle θb2. The angle θb2 is a known value, and the lengthb2 canbe calculated, for example, using predetermined coefficients and based on b and c . With a s imi lar method, the partial area Sc1 and the partial area Sc2 are determined. The partial area Sc1 is calculated from c, c1, and θc1, and the partial area Sc2 is calculated from c1, c2, and θc2. Because angles θc1 and θc2 are known, c1 and c2 may be estimated based on c or based on c and b. Finally, an area S in which the partial areas Sb, Sc, Sb1, Sb2, Sc1, and Sc2 are added is determined. The area S is output as an index value representing the amount of visceral fat, or an index value is determined by converting or correcting the area S. In either case, the size of the visceral fat area 20 is measured from a two-dimensional viewpoint, so that an index value can be obtained with higher reliability than the measurement of the abdominal circumference (that is, the method of the related art).

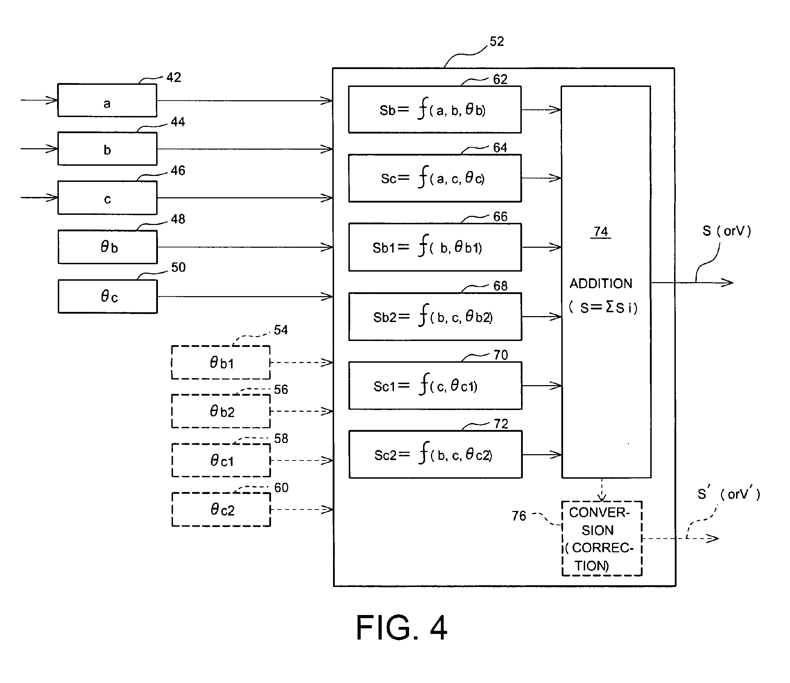

FIG. 4 is a conceptual diagram showing the above-described method. Reference numeral 52 represents a calculation module. The calculation at the calculation module is realized by, for example, a function of software. Numerical values a, b, c, θb, and θc represented with reference numerals 42 - 50 are input to the module 52. In addition, values of θb1, θb2, θc1, and θc2 represented by reference numerals 54 - 60 are supplied as necessary. Based on these parameter values, as shown by reference numerals 62 - 72, six partial areas Sb - Sc are calculated by the functions shown in the figure. The exemplified configuration is merely one example, and, in any configuration, desirably, the three measured distances a, b, and c and the two angles θb and θc which are known are used. Reference numeral 74 represents an addition of the 6 partial areas. The area S which is the added result may be output as the index value or a volume may be calculated based on the plurality of areas and output. Moreover, in the calculation of the evaluation value, the body constitution, age, sex, or the like of the subject may be considered, which is shown with reference numeral 76. Specifically, a correction is applied based on various conditions on S which is the added result, and a final index value S' may be determined. The index value may be a volume V'. When the volume is determined, areas are determined at a plurality of positions on the living body and the volume is desirably determined based on these areas. Alternatively, if the volume can be determined from the area based on experience, such a conversion may be executed.

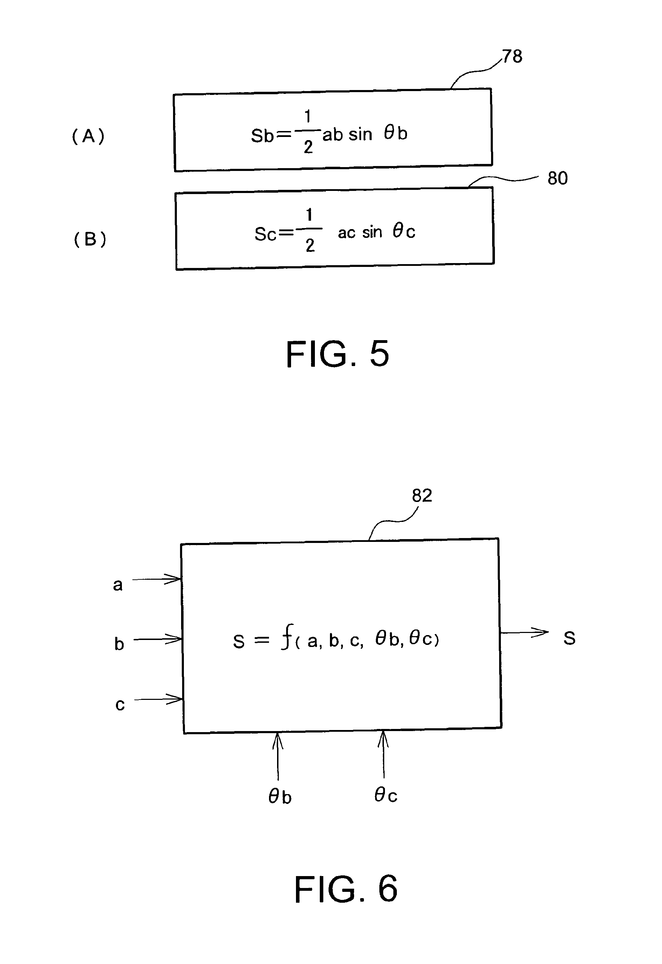

FIG. 5 exemplifies a basic calculation equation of two partial areas. Reference numeral 78 shows a calculation method of the partial area Sb. That is, a calculation of 1/2·ab sin θb is executed. Reference numeral 80 shows a calculation method of the partial area Sc. That is, a calculation of 1/2·ac sin θc is executed.

As shown in FIG. 6, a table 82 may be employed in which a, b, c, θb, and θc are supplied as input values and S is determined as an output value. For example, data may be obtained from many subjects and may be accumulated, in order to construct such a table.

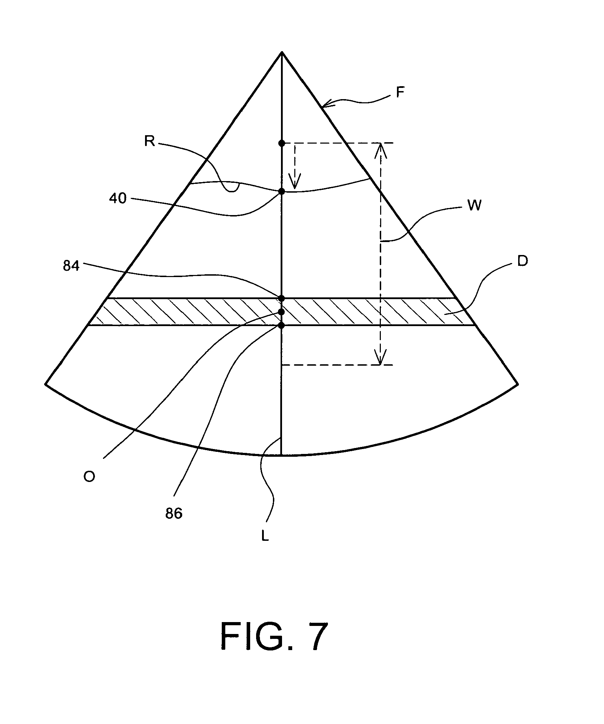

FIG. 7 shows an automatic calculation method of the distance. On a frame F, a measurement line L is set as a central line. W represents a search range. For example, by executing an edge detection process from an origin of the search range W, it is possible to identify an edge point 40 on the boundary surface R. The search direction may be toward the upward direction. Alternatively, a bloodstream section D may be extracted using an ultrasonic Doppler method, and, based on a result of such an image process, two edge points 84 and 86 may be identified, and a center point O may be identified as an intermediate point of the edge points. The bloodstream section may be identified without the use of the Doppler information and by a binarization process or the like. With such an automatic calculation, the burden of the user can be significantly reduced. The effectiveness of the automatic calculation can be recognized in particular for a group medical examination or the like.

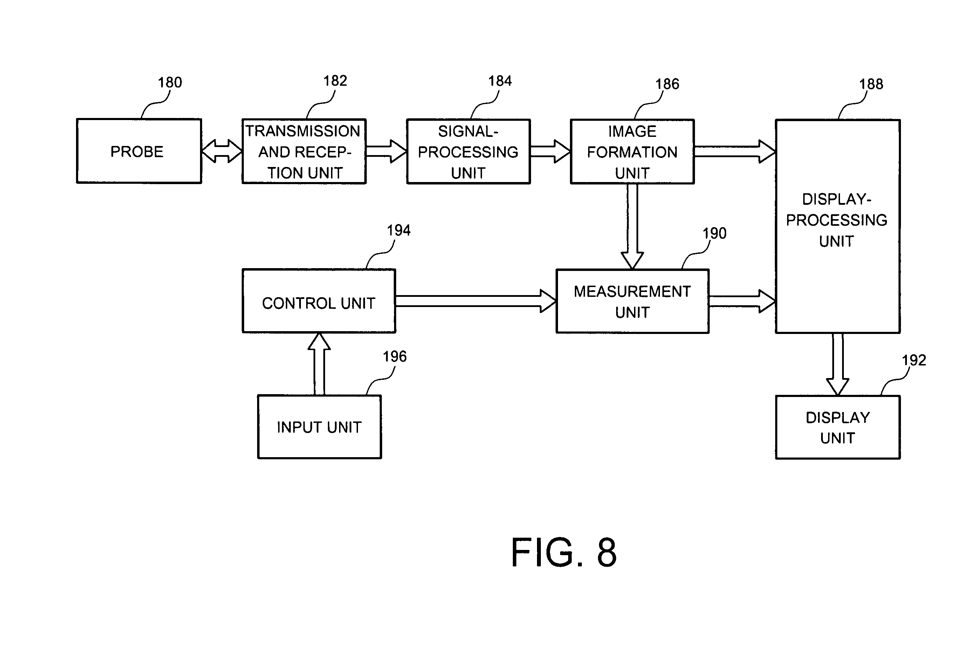

FIG. 8 is a block diagram showing an ultrasonic diagnostic device having the above-described function to calculate the index value.

A probe 180 is connected to a body through a cable, and in the present embodiment, the probe 180 comprises a 1-D (one-dimensional) array transducer. The 1-D array transducer is formed from a plurality of transducer elements which are arranged in a linear shape or an arc shape. An ultrasonic beam is formed by the array transducer. The ultrasonic beam is electrically scanned. As such a scanning method, an electric linear scanning, electronic sector scanning, etc. are known. In the present embodiment, an arc-shaped array transducer is used, and a scanning method which is known as convex scanning is executed. A single probe 180 is used, and the same probe is sequentially brought into contact at the plurality of contact positions in multiple stages.

A transmission and reception unit 182 functions as a transmission beam former and a reception beam former. During transmission, the transmission and reception unit 182 supplies a plurality of transmission signals inparallel to the array transducer. With this process, a transmission beam is formed at the probe 180. A reflected wave from the inside of the living body is received by the probe 180, and a plurality of reception signals are output to the transmission and reception unit 182. During reception, the transmission and reception unit 182 executes a phase align and summing process on the plurality of reception signals to form a reception signal after the phase align and summing, and outputs beam data. The beam data is supplied to a signal-processing unit 184. The signal-processing unit 184 comprises a logarithmic converter, a wave detector, etc.

The beam data after the signal process are supplied to an image formation unit 186. The image formation unit 186 is formed from a digital scan converter, which includes a coordinate conversion function and an interpolation process function. A B-mode black-and-white tomographic image is formed by a plurality of sets of beam data. The image data are supplied to a display-processing unit 188. A tomographic image is displayed on a display unit 192.

A measurement unit 190 is a module which executes automatic distance measurement or a module which executes a distance calculation based on a position which is manually input. A control unit 194 executes operation control of the structures shown in FIG. 1. The control unit 194 is formed from a CPU and an operation program. An input unit 196 is formed from an operation panel or the like, and more specifically, comprises a keyboard, a trackball, etc. The user can designate a position by means of the input unit 196.

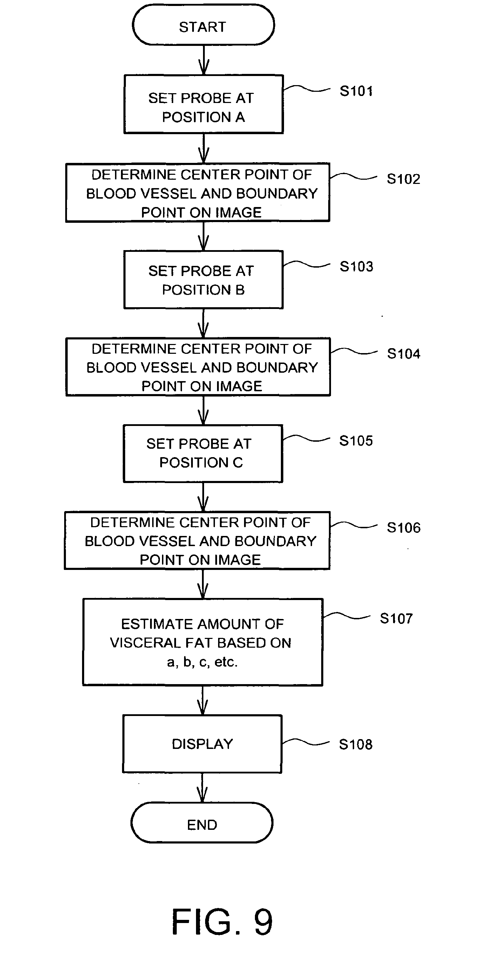

FIG. 9 shows an operation of the device shown in FIG. 8. In particular, FIG. 9 shows an operation when the index value is calculated.

In a state where the living body lies on a bed facing upward, the holding equipment is placed on the abdominal region. Then, the probe is set at a position A in S101. The position A is, for example, the central position. In S102, on a tomographic image formed using the probe thus placed, a center point of the blood vessel and the boundary point are input by the user, and a distance therebetween is measured. Prior to this process, the orientation of the probe is adjusted by the user so that a desired cross section is drawn. This process corresponds to a process of setting the central measurement line. The holding equipment is formed with a soft material to permit such an inclining movement; that is, the raking movement.

When the distance measurement is completed at the central position, in S103, the probe is set at a position B, and, in S104, the orientation and position of the probe are adjusted and the center of the blood vessel and the boundary point are designated by the user on a B-mode tomographic image. With this process, a second distance is measured. Similarly, in S105, the probe is set at a position C, and, in S106, the position and the orientation of the probe are adjusted and the center point of the blood vessel and the boundary point are designated by the user on an ultrasonic image. With this process, a third distance is measured.

In S107, based on the three distances and two angles which are defined in advance, an index value having a high correlation to the amount of visceral fat is calculated. The calculation corresponds to estimation of the amount of visceral fat. In S108, the index value is displayed. In a group medical examination, by not simply measuring the abdominal circumference, but also estimating the visceral fat existing area in the abdominal cavity using the ultrasonic diagnosis and in a two-dimensional shape as described above, it becomes possible to obtain a more useful index value for diagnosing or evaluating metabolic syndrome. In order to further improve the correlation between the index value and the amount of visceral fat, in the diagnosis, it is desirable to apply correction based on the sex, physical constitution, and other information pertaining to the subject. A coefficient used for such a correction is determined based on experience or experimentally.

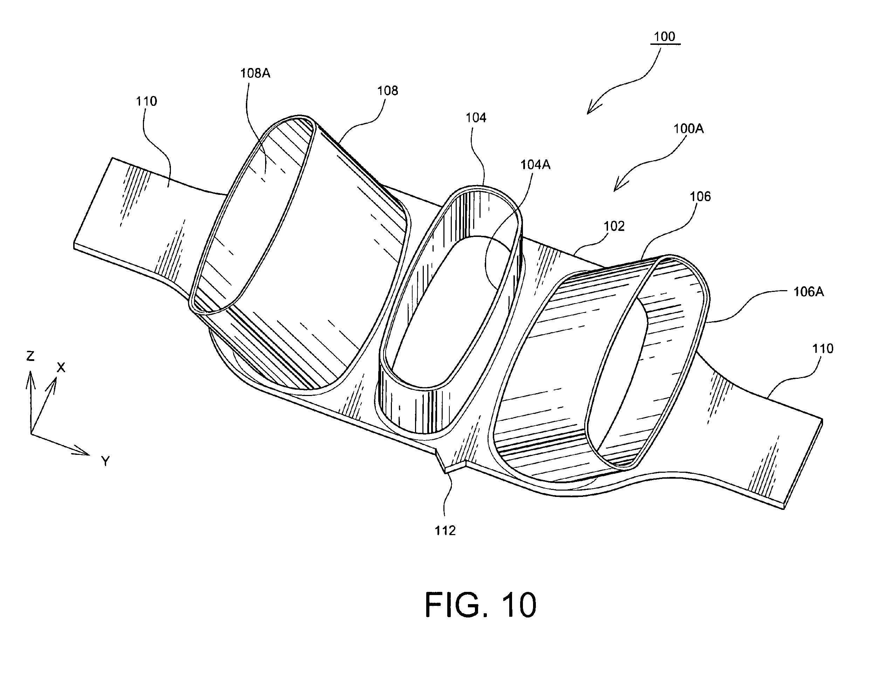

Next, with reference to FIGs. 10 - 12, the holding equipment used for the measurement of the index value as described above will be described in detail. The holding equipment may be used for usages other than the above-described measurement.

FIG. 10 shows a structure of primary portions of the holding equipment 100. Reference numeral 100A represents a body unit. The body unit 100A has a base 102, and three holding portions 104, 106, and 108 are provided on the base 102. The holding portions 104, 106, and 108 have hollow portions 104A, 106A, and 108A, respectively, where the probe is inserted and is gently held. A horizontal cross sectional shape of the hollow portions 104A, 106A, and 108A is uniform along a depth direction. Alternatively, the shape may be deformed along an outer shape of the probe.

The body unit 100A is formed from a soft material such as, for example, rubber, and a belt section 110 connected to the body unit 100A is also formed from rubber or the like. However, when the belt section 110 is used to measure the abdominal circumference or the like, the belt section 110 is desirably formed from a material which is not stretchable. By providing the belt section 110, it becomes possible to fix the body unit 100A while the surroundings of the abdominal region are enwrapped by the belt section 110, and the probe can be easily held in a stable state. With such a configuration, an orthogonal coordinate system referencing the subject can be easily defined. In addition, with the use of such holding equipment 100, the probe can be followed and moved with the surface movement of the living body during respiration, a problem such as position deviation for measurement can be reduced, and reproducibility of the measurement can be improved.

Because three probe positions are defined in the measurement of the index value as described above, the body unit 100A shown in FIG. 10 has three holding portions 104, 106, and 108. The holding portion 104 at the center stands vertically; that is, has a vertical orientation extending in the Z direction. The two other holding portions 106 and 108 are provided with an inclination angle of a predetermined angle; more specifically, 40 degrees, with respect to the holding portion 104. These holding portions are inclined in the YZ plane. Because the body unit 100A itself is made from a soft material, the probe inserted in each holding portion can be inclined. Movement of the probe itself is permitted in the direction in the YZ plane, and movement of the probe in other directions is limited. With this configuration, the search of the descending aorta can be easily executed, and three scanning planes can be accurately crossed on the descending aorta. Reference numeral 112 shows a navel marker, and a projected portion of the marker is matched with the position of the navel. With this configuration, reproducibility of measurement and superiority of ultrasound propagation can be ensured.

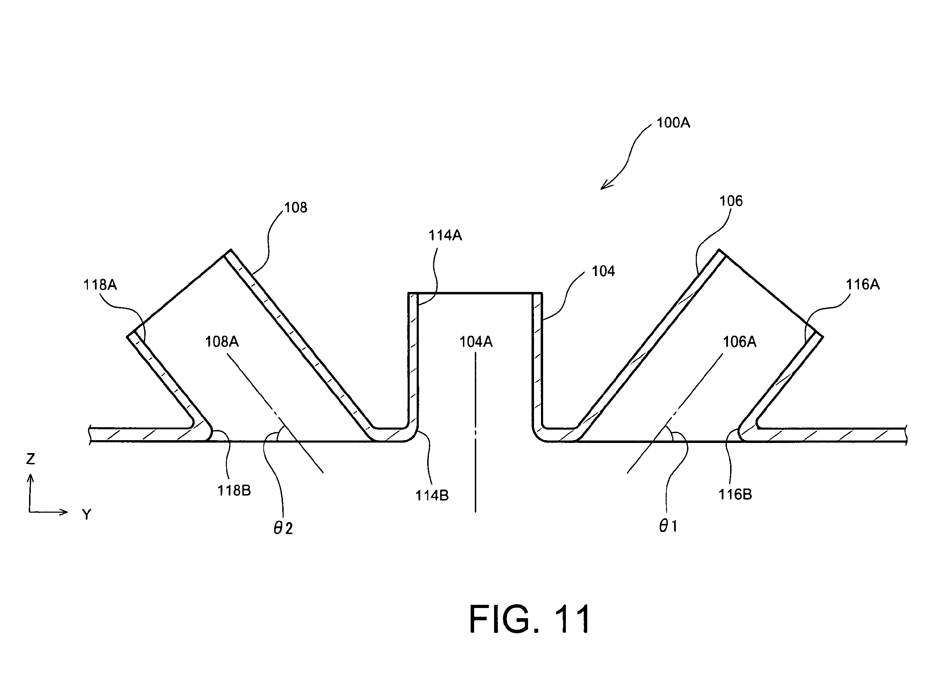

FIG. 11 shows a cross section of the body unit 100A. As described above, the three holding portions 104, 106, and 108 are hollow structures, and are radially aligned. In FIG. 11, an angle θ1 is, for example, 40 degrees, and an angle θ2 is, for example, also 40 degrees. Alternatively, so long as the suitable angle can be realized during equipment, in the original form state, the angles θ1 and θ2 may be smaller angles. The central holding portion 104 stands vertically. The hollow insides 104A, 106A, and 108A have shapes to just enfold the outer side of the probe, and, when the probe is held, the probe may be inclined due to the degree of freedom of movement in the hollow inside and the elasticity of the body, but the probe does not easily drop from the holding portion. Reference numeral 114A represents an upper opening, and reference numeral 114B represents a lower opening. Similarly, reference numeral 116A represents an upper opening, reference numeral 116B represents a lower opening, reference numeral 118A represents an upper opening, and reference numeral 118B represents a lower opening. Ina state where the probe is set, it is desirable that the transmission and reception surface; more specifically, an acoustic lens surface, of the probe closely contacts the body surface, and, in order to remove an air layer between the transmission and reception surface and the living body surface, for example, an acoustic coupling member of jelly form is used. The body unit 100A may be formed from a transparent material in order to ensure visibility.

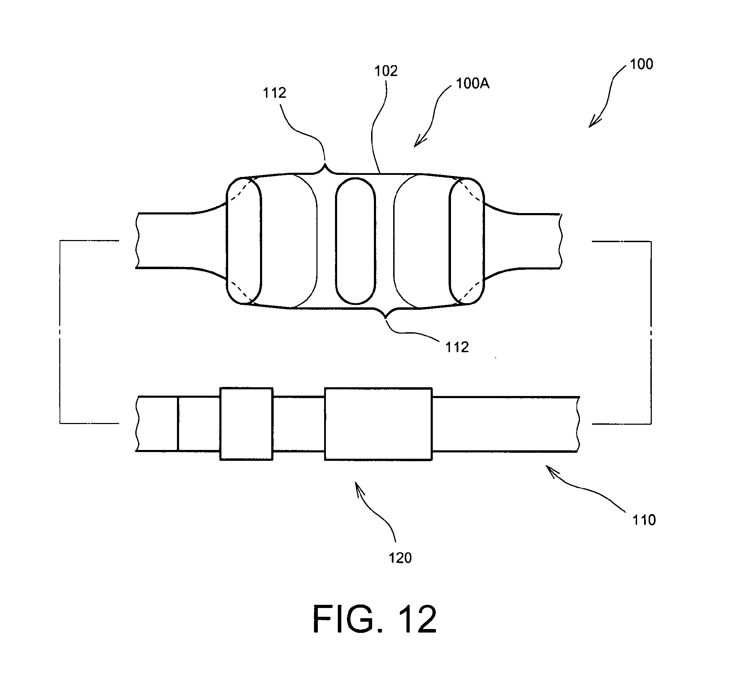

FIG. 12 conceptually shows the entirety of the holding equipment 100. As described above, the holding equipment 100 includes the body unit 100A, and the belt section 110 connected on both ends of the body unit 100A. The belt section 110 is to be wound around the body section of the living body. The belt section 110 itself may be stretchable, or, for example, a mechanism having an adjustable length may be provided as shown with reference numeral 120. In addition, in order to allow measurement of the abdominal circumference during length adjustment, scaling marks may be provided on the belt section 110. On both sides of the body unit 100A including one side and the other side, the protruded marker 112 is provided so that the marker 112 can be matched with a position of the navel regardless of the orientation the body unit 100A is placed in, and a problem such as re-doing the placement of the holding equipment 12 or the like can be prevented in advance. With the employment of the navel marker 112, the probe contact position can always be shifted from the center of the living body to one side by a predetermined distance, which allows both formation of superior propagation path and superior reproducibility of measurement. Such a position corresponds to a position above the descending aorta, and the vertical positioning can be executed simultaneously.

Because the holding equipment shown in FIGs. 10 - 12 facilitates setting of the plurality of scanning planes in a state of intersecting each other on a reference site existing in a deep portion within a living body, the holding equipment can be generally used in cases, in addition to the calculation of the index value described above, where similar measurement is desired. The elasticity or the degree of holding action of the holding equipment may be adjusted according to the usage. With the use of such holding equipment, in a group medical examination, a doctor can easily complete positioning of the probe by merely sequentially inserting the probe in the holding portions, and, thus, the burden of the doctor can be significantly reduced. In addition, because the reproducibility of measurement can be significantly improved, the efficiency of the group medical examination can be improved.

In the present embodiment, the index value is measured in a state where the living body faces a lateral direction. Alternatively, the index value may be measured using similar holding equipment in a state where the living body is standing.

## Claims

1. An ultrasonic diagnostic system comprising:
an ultrasonic probe which is brought into contact with a living body, which transmits and receives ultrasound, and which outputs a reception signal;
an image formation unit which forms an ultrasonic image based on the reception signal;
a distance measurement unit which uses a plurality of ultrasonic images including a plurality of measurement paths which are radially set on a cross section of the living body, to measure a distance between a reference site at a deep position and a predetermined boundary surface at a shallow position along each of the measurement paths;
an index value calculation unit which calculates an index value having a correlation to an amount of visceral fat based on a relative positional relationship among the plurality of measurement paths and a plurality of distances measured along the plurality of measurement paths; and
an output unit which outputs the index value.

2. The ultrasonic diagnostic system according to Claim 1, wherein the reference site is a blood vessel,
each ultrasonic image corresponding to each of the measurement paths is displayed as a tomographic image, and
a distance between the blood vessel and the predetermined boundary surface is measured on each of the tomographic images.

3. The ultrasonic diagnostic system according to Claim 2, wherein the blood vessel is a descending aorta which beats.

4. The ultrasonic diagnostic system according to Claim 3, wherein
the plurality of tomographic images correspond to a plurality of scanning planes which are perpendicular to the cross section and which cross each other on the descending aorta.

5. The ultrasonic diagnostic system according to Claim 4, wherein
the cross section of the living body is a lateral cross section on an abdominal region of the living body, and
a central scanning plane among the plurality of scanning planes is formed at a position avoiding a navel existing in the abdominal region.

6. The ultrasonic diagnostic system according to Claim 5, wherein
the plurality of scanning planes include a central scanning plane, a right-side scanning plane, and a left-side scanning plane, and
the right-side scanning plane and the left-side scanning plane are set on a right side and a left side of the central scanning plane with substantially the same inclination angle with respect to the central scanning plane.

7. The ultrasonic diagnostic system according to Claim 1, wherein
the plurality of measurement paths include a central path, a right-side path, and a left-side path, and
the index value calculation unit comprises:
a unit which calculates a right-side portion area of a right-side portion between the central path and the right-side path based on a distance along the central path, a distance along the right-side path, and a right-side angle between the central path and the right-side path;
a unit which calculates a left-side portion area of a left-side portion between the central path and the left-side path based on the distance on the central path, a distance along the left-side path, and a left-side angle between the central path and the left-side path; and
a unit which calculates the index value using at least the right-side portion area and the left-side portion area.

8. The ultrasonic diagnostic system according to Claim 1, wherein
the predetermined boundary surface is an inner surface of a subcutaneous layer.

9. The ultrasonic diagnostic system according to Claim 1, wherein
each of the ultrasonic images includes a line representing a corresponding measurement path, and
the system further comprises an input unit with which a user designates the reference site and the predetermined boundary surface on each of the lines.

10. The ultrasonic diagnostic system according to Claim 1, further comprising:
probe-holding equipment for maintaining a state where the probe is brought into contact with a surface of an abdominal region of the living body.

11. The ultrasonic diagnostic system according to Claim 10, wherein the probe-holding equipment comprises:
a plurality of holding portions which store a probe to be brought into contact with the abdominal region; and
a fixing unit which fixes the plurality of holding portions on the abdominal region, and
the plurality of holding portions are provided aligned in a left-and-right direction and with an angle to direct a transmission and reception surface of the probe toward the reference site during use.

12. The ultrasonic diagnostic system according to Claim 11, wherein
each of the holding portions has deformability to permit a raking movement of the probe stored therein.

13. The ultrasonic diagnostic system according to Claim 11, wherein
the probe includes a one-dimensional array transducer, and
eachof the holding portions holds the probe such that an element arrangement direction of the one-dimensional array transducer is parallel to a body axis direction of the living body.

14. The ultrasonic diagnostic system according to Claim 11, wherein
in each of the holding portions, there are formed an opening for exposing the transmission and reception surface of the probe to the living body side and a hollow structure which surrounds and holds the probe.

15. The ultrasonic diagnostic system according to Claim 11, wherein
the plurality of holding portions include a central holding portion, a right-side holding portion, and a left-side holding portion;
the right-side holding portion and the left-side holding portion are inclined with respect to the central holding portion, and the overall holding portions spread in a fan shape, and
inclination angles of the right-side holding portion and the left-side holding portion with respect to the central holding portion during use are set between 30 degrees and 50 degrees.

16. The ultrasonic diagnostic system according to Claim 11, wherein
the fixing unit is a belt-shaped member wound around the body section.

17. The ultrasonic diagnostic system according to Claim 11, wherein
a marker which is used for position matching with respect to a navel is provided.
